# FASCICULE DE BREVET EUROPEEN

(11) **EP 0 404 660 B1**
(45) Date de publication et mention de la délivrance du brevet: **15.05.1996**
(21) Numéro de dépôt: 90401722.5
(22) Date de dépôt: 19.06.1990
(51) Int. Cl.: A61K 38/00

(54) **Utilisation de complexes glycoprotéines extraits de bactéries gram (-) pour la fabrication d'un médicament facilitant la cicatrisation de la peau et procédé de préparation**
Verwendung von Glykoproteinkomplexextrakten aus Gram (-)-Bakterien zur Herstellung eines Arzneimittels zur Erleichterung der Vernarbung der Haut und sein Herstellungsverfahren
Use of glycoprotein complex extracts of gram (-) bacteria for the preparation of a medicament to facilitate the cicatrisation of the skin and a method for its preparation

(30) Priorité: 20.06.1989 FR 8908194
(43) Date de publication de la demande: 27.12.1990
(73) Titulaire: ROUSSEL-UCLAF, F-75007 Paris (FR)
(72) Inventeur: Bonfils, Armelle, F-78700 Conflans Sainte Honorine (FR); Smets, Pierre, F-78670 Villennes Sur Seine (FR); Zalisz, René, F-95180 Menucourt (FR)
(74) Mandataire: Jacobi, Markus Alexander

(56) Documents cités:
- FR-A- 2 315 945
- FR-A- 2 378 855
- FR-A- 2 405 298
- FR-A- 2 574 429
- GB-A- 2 157 566
- PHLEBOLOGIE, vol. 36, no. 2, 1983, pages 203-208, Paris, FR; G. MEGRET: "Une nouvelle thérapeutique locale dans le traitement des ulcères de jambe: le biostim crème"

## Description

La présente invention a pour objet l'utilisation de complexes glyco-protéiques extraits de bactéries gram (-) pour la fabrication d'un médicament pour le traitement de l'alopécie ainsi que le procédé de préparation de ces complexes.

Dans la demande française n° 2574429, la demanderesse a décrit des acylglycannes extraits de corps microbiens lysés de Klebsiella pneumoniae, le procédé de préparation et l'application à titre de médicament anti-allergique et immunomodulateur de cette nouvelle fraction.

Dans PHLEBOLOGIE (vol. 36, 2, 1983, 203-208) l'utilisation d'un produit qui contient des glycoprotéines extraites de Klebsiella pour le traitement de l'ulcère de jambe est mentionnée.

En poursuivant ses études sur les produits précités, la demanderesse vient de trouver de façon tout à fait inattendue que des fractions provenant d'un procédé de préparation plus rapide présentaient de très remarquables propriétés dans le domaine du traitement de l'alopécie.

Parmi les complexes glyco-protéiques extraits de bactéries gram (-), on retient notamment les complexes glycoprotéiques extraits de Klebsiella pneumoniae.

Selon l'invention, les complexes glyco-protéiques possèdent de remarquables propriétés cicatrisantes dans le traitement de l'alopécie.

L'invention a ainsi pour objet l'utilisation des complexes glycoprotéiques tels que définis ci-dessus pour la fabrication d'un médicament facilitant la cicatrisation cutanée dans le traitement de l'alopécie.

Parmi les complexes glycoprotéiques extraits de Klebsiella pneumoniae on retient de préférence les complexes glyco-protéiques extraits de la souche de Klebsiella pneumoniae déposée à l'Institut Pasteur à Paris sous le n° I-163.

En étudiant l'activité des complexes glycoprotéiques de la présente demande, on a constaté que ceux-ci présentaient une remarquable activité inductrice "like" de l'EGF (Epidermal Growth Factor) facteur de croissance épidermique sans présenter la toxicité de ce dernier. De ce fait les complexes glycoprotéiques peuvent être utilisés pour faciliter la cicatrisation de la peau et de l'épiderme.

En raison de leurs remarquables propriétés cicatrisantes illustrées plus loin dans la partie expérimentale, les complexes glycoprotéiques selon l'invention, peuvent être utilisés dans le traitement des brûlures de toutes origines, dans le traitement des grands brûlés, des érythèmes solaires, des plaies superficielles, des gerçures, des crevasses, des engelures, des piqûres d'insectes, en chirurgie ou en chirurgie plastique, pour faciliter la cicatrisation, dans le vieillissement cutanée, enfin en alopécie.

La dose usuelle, variable selon le produit utilisé, le sujet traité et l'affection en cause, peut être, par exemple de 50 µg à 5 mg par jour en applications locales chez l'homme.

A titre de médicaments, les complexes glyco-protéiques de la présente demande peuvent être administrés en applications locales.

Les compositions pharmaceutiques correspondantes peuvent être, par exemple, solides ou liquides et se présenter sous les formes pharmaceutiques couramment utilisées en médecine humaine, comme par exemple, les crèmes, les gels, les pommades, les lotions, les laits ou huiles pour la peau, les gouttes, les collyres, les aérosols, les shampooings ou sous forme de liposomes ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Le complexe glycoprotéique selon l'invention peut être préparé par un procédé qui consiste à cultiver sur un milieu solide ou liquide, une souche de bactéries gram (-), à récolter après complet développement les corps microbiens, à procéder à une lyse de ces derniers, à recueillir le lysat, à traiter ce dernier au moyen d'un ou de plusieurs solvants organiques et à recueillir le produit brut résultant, procédé caractérisé en ce que l'on met ce dernier en solution aqueuse, centrifuge, recueille le surnageant et lyophilise.

Dans le procédé ci-dessus décrit, la lyse des corps microbiens est avantageusement une lyse enzymatique, le traitement du lysat au moyen d'un ou de plusieurs solvants organiques est avantageusement réalisé en effectuant successivement un traitement au moyen de l'acétone et du méthanol. Selon une variante du procédé le surnageant provenant de l'extraction au moyen d'un ou de plusieurs solvants organiques peut également être précipité au moyen de l'éthanol puis être centrifugé avant d'être remis en solution aqueuse puis être lyophilisé.

Le procédé de préparation de l'extrait brut de Klebsiella pneumoniae a été décrit dans la demande de brevet français n° 2574429.

Il va être donné maintenant à titre non limitatif des exemples de mise en oeuvre de l'invention.

### EXEMPLE 1 : complexe glycoprotéique extrait de Klebsiella pneumoniae

L'extrait acétone/méthanol lyophilisé obtenu au stade C de l'exemple 1 de la demande de brevet français n° 2574429 est remis en solution dans l'eau distillée à la concentration de 20 g/litre et placé sous agitation pendant 16 h à 4°C. La solution est centrifugée à 14 000 g pendant 30 minutes à 15°C. Le surnageant est lyophilisé pour obtenir le complexe glycoprotéique de Klebsiella pneumoniae.

On obtient 20 g/l de fermenteur de complexe glycoprotéique extrait de Klebsiella pneumoniae (souche I.163 déposée à l'Institut Pasteur de Paris).

### EXEMPLE 2 : complexe glycoprotéique extrait de Klebsiella pneumoniae

L'extrait acétone/méthanol lyophilisé obtenu au stade C de l'exemple 1 de la demande de brevet français n° 2574429 est remis en solution dans l'eau distillée à la concentration de 20 g/litre et placé sous agitation pendant 16 h à 4°C. La solution est ensuite centrifugée à 14 000 g pendant 30 minutes à 15°C. Le surnageant est précipité par 3 volumes d'éthanol absolu et centrifugé à 14 000 g pendant 30 minutes à 4°C.

Le culot est alors remis en solution dans de l'eau distillée et lyophilisé. On obtient 10 g/l de fermenteur de complexe glyco-protéique extrait de Klebsiella pneumoniae (souche I.163 déposée à l'Institut Pasteur de Paris).

### EXEMPLE 3 : pommade cicatrisante

On a préparé une pommade cicatrisante répondant à la formule :

| | |
|---|---|
| - complexe glycoprotéique extrait de Klebsiella pneumoniae (exemple 1) | 0,5 g |
| - excipient q.s.p. | 100 g |

### ETUDE DE L'ACTIVITE CICATRISANTE

### Culture

Des fibroblastes de derme humain sont multipliés en culture monocouche dans du Milieu de Eagle avec sels de Earle (EMEM, Laboratoires Boeringher) contenant 10 % de sérum de veau foetal (Laboratoires Flow). Les expériences sont effectuées avec des cultures variant de 7 à 10 passages.

Des gels tridimentionnels de 30 ml sont réalisés, en mélangeant 13,8 ml de EMEM (1,76 fois concentré), 2,7 ml de sérum de veau foetal ou de EMEM (selon les expériences), 9 ml de collagène acido-soluble extrait à partir de tendons de queues de rats (3 mg/ml dans de l'acide acétique 1/1000), 1,5 ml de NaOH 0,1N et 3 ml de suspension de fibroblastes dans du EMEM (4 x 10⁵ cellules/ml). Ce mélange est placé à 37°C dans des boîtes de Pétri de bactériologie de 100 mm de diamètre pour permettre la polymérisation du collagène et la formation d'un gel dans lequel les fibroblastes sont répartis en trois dimensions.

Des biopsies circulaires de 1 mm de diamètre sont prélevées dans des fragments de peau saine provenant de chirurgie réparatrice (plasties mammaires de femmes âgées de 20 à 45 ans). Selon les expériences ces biopsies sont implantées au centre des dermes équivalents dès la formation du gel, ou collées par une goutte de collagène sur des dermes dont la contraction est stabilisée (au 5ème jour). Dans le dernier cas, le collage de l'implant est réalisé soit sur dermes vivants, soit sur dermes dont les fibroblastes sont tués par choc osmotique avant l'épidermisation.

### Traitement par le complexe glycoprotéique

Le complexe glycoprotéique étudié est dissout au moment du traitement dans du sérum de veau foetal, de façon qu'il soit à une concentration finale dans la culture de 0,0005 ‰, 0,005 ‰, 0,05 ‰, 0,5 ‰ et 5 ‰.

Le complexe glycoprotéique est ajouté à partir du 5ème jour, une fois que les fibroblastes ont remanié la matrice de collagène, et donc que le traitement par le complexe glycoprotéique n'a plus d'effet sur cette fonction.

La durée totale du traitement est de 13 jours. Le milieu est renouvelé, avec ou sans complexe glycoprotéique deux fois par semaine, en prenant garde à ce que les cultures soient toujours en immersion. Dans toutes les expériences les différents échantillons sont en 3 exemplaires.

### Evaluation quantitative de l'épidermisation

A la fin de chaque expérience, les peaux équivalentes sont transférées dans des boites de Pétri de 60 mm de diamètre, incubées 1 heure à 37°C dans 3 ml de milieu de culture contenant 2 µCi/ml de thymidine tritiée (6⁻³ H Thymidine, 25 Ci/mM, CEA). Puis les spécimens sont incubés 30 min. à 37°C dans du sulfate de Bleu du Nil (Sigma) à la concentration finale de 1/10000, et rincés 15 min. à la température du laboratoire dans du NaCl 9 ‰, ce qui permet de bien visualiser l'épiderme qui s'est développé à la surface du derme équivalent à partir de l'implant initial.

La surface épidermisée est alors mesurée par planimétrie à l'aide d'un analyseur d'images (Nachet NS 1000).

L'étude est effectuée comparativement avec des cultures en absence et en présence d'EGF (20 mg/ml).

Les résultats figurant dans le tableau I ci-après sont la moyenne de 3 déterminations. Ces résultats montrent que les complexes glycoprotéiques tels que définis ci-dessus sont de puissants facteurs de croissance, souvent plus efficaces que l'EGF dans la stimulation de la croissance épidermique.

**TABLEAU I**

| Surface en mm₂ | | | | | |
|---|---|---|---|---|---|
| Produit étudié | Contrôle sans EGF | Contrôle EGF 20 mg/ml | Complexe 0,5 ug/ml | glyco-protéique | |
| | | | | 5 ug/ml | 50 ug/ml |
| - | 36,34 | 379,77 | | | |
| Ex. 2 | | | 31,53 | 142,00 | 413,00 |
| - | 16,20 | 192,23 | | | |
| Ex. 1 | | | 40,15 | 73,80 | 133,63 |

## Revendications

1. Utilisation de complexes glycoprotéiques extraits de la souche de la bactérie Klebsiella pneumonica, déposée à l'Institut Pasteur à Paris sous le no. I-163, préparés par un procédé qui consiste
a) à cultiver sur un milieu solide ou liquide la souche de bactéries,
b) à récolter les corps microbiens,
c) à procéder à une lyse de ces derniers,
d) à recueillir le lysat,
e) à traiter ce dernier au moyen d'un ou de plusieurs solvants organiques,
f) à recueillir le produit brut résultant,
g) à mettre ce dernier en solution aqueuse,
h) à centrifuger cette solution,
i) à recueillir le surnageant et
g) à lyophiliser ce produit,
pour la fabrication d'un médicament pour le traitement de l'alopécie.

## Claims

1. Use of glycoprotein complexes extracted from the strain of the Klebsiella pneumonica bacterium, deposited at the Pasteur Institute in Paris under No. I-163, prepared according to a process which consists of:
a) cultivating on a solid or liquid medium the strain of bacteria,
b) collecting the microbial bodies,
c) proceeding with lysis of the latter,
d) collecting the lysate,
e) treating the latter by means of one or more organic solvents,
f) collecting the resulting crude product,
g) putting the latter into aqueous solution,
h) centrifuging this solution,
i) collecting the supernatant and
j) lyophilizing this product,
for the production of a medicament for the treatment of alopecia.

## Patentansprüche

1. Verwendung von Glykoproteinkomplex-Extrakten des Bakterienstammes Klebsiella pneumonica, hinterlegt am Institut Pasteur in Paris unter der Nummer I-163, hergestellt durch ein Verfahren, das besteht aus
a) Kultivierung des Bakterienstammes in einem festen oder flüssigen Milieu,
b) Gewinnung der mikrobiellen Körper,
c) Durchführung einer Lyse mit diesen Körpern,
d) Sammeln des Lysates,
e) Behandlung dieses Lysates mit Hilfe von einem oder mehreren organischen Lösungsmitteln,
f) Gewinnung des daraus resultierenden rohen Produktes,
g) Überführung dieses Produktes in eine wäßrige Lösung,
h) Zentrifugieren dieser Lösung,
i) Gewinnung des aufschwimmenden Anteiles, und
j) Lyophilisierung dieses Produktes,
zur Herstellung eines Medikamentes für die Behandlung von Alopezie.
